# EUROPÄISCHE PATENTSCHRIFT

(11) **EP 1 351 902 B1**
(45) Veröffentlichungstag und Bekanntmachung des Hinweises auf die Patenterteilung: **14.05.2008**
(21) Anmeldenummer: 02708258.5
(22) Anmeldetag: 05.01.2002
(51) Int. Cl.: C07C 6/08

(54) **VERFAHREN ZUR HERSTELLUNG VON KOHLENWASSERSTOFFEN MIT VERÄNDERTEM KOHLENSTOFFGERÜST**
METHOD FOR PRODUCING HYDROCARBONS HAVING A MODIFIED CARBON SKELETON
PROCEDE DE PRODUCTION D'HYDROCARBURES A SQUELETTE CARBONE MODIFIE

(30) Priorität: 08.01.2001 DE 10100485
(43) Veröffentlichungstag der Anmeldung: 15.10.2003
(73) Patentinhaber: BASF SE, 67056 Ludwigshafen (DE)
(72) Erfinder: VANOPPEN, Dominic, 2950 Kapellen (BE); SCHWAB, Ekkehard, 67434 Neustadt (DE); BASSET, Jean-Marie, F-69300 Caluire (FR); THIVOLLE-CAZAT, Jean, F-69270 Fontaine sur Saône (FR); TAOUFIK, Mostapha, F-69140 Rillieux La Pape (FR); SCHULZ, Michael, BASF East Asia Regional, No.1 Connaught Place, Central, Hong Kong (CN); HÖHN, Arthur, 67281 Kirchheim (DE)
(86) Internationale Anmeldenummer: PCT/EP2002/000054
(87) Internationale Veröffentlichungsnummer: WO 2002/053520

(56) Entgegenhaltungen:
- WO-A-98/02244
- GB-A- 858 649
- US-A- 5 414 184
- SARKANY, A., TETENYI, P: "Homologation of Pentanes and Hexanes on Ni Catalysts" JOURNAL OF THE CHEMICAL SOCIETY, CHEMICAL COMMUNICATIONS, 1980, Seiten 525-527,
- SARKANY, A.: "Homologation of Small Alkanes on Pt, Pd and Ni Catalysts" JOURNAL OF THE CHEMICAL SOCIETY, FARADAY TRANSACTIONS I, Bd. 82, 1986, Seiten 103-108,
- O'DONOHOE, C., CLARKE, J.K.A., ROONEY, J.J.: "Homologation of n-Alkanes on Tungsten Films. A Novel, Highly Selective Reaction of Intermediate Carbenes" JOURNAL OF THE CHEMICAL SOCIETY, CHEMICAL COMMUNICATIONS, 1979,

## Beschreibung

Die vorliegende Erfindung betrifft ein Verfahren zur Herstellung von Kohlenwasserstoffen mit verändertem Kohlenstoffgerüst in Gegenwart von Wasserstoff.

Aus der WO-A-98/02244 ist ein Verfahren zur Veränderung von aliphatischen Kohlenwasserstoffketten bekannt. Als Katalysatoren werden Materialien beschrieben, die als aktives Zentrum das Hydrid eines Elementes der Gruppen 5 oder 6 des periodischen Systems der Elemente auf einem festen Trägers enthalten. Bereits bei der einfachen Disproportionierung von Propan ist die Aktivität des Katalysatorsystems sehr gering. So werden hierbei innerhalb von 43 Stunden lediglich 43 katalytische Zyklen je Aktivzentrum durchlaufen.

Aus der WO-A-00/27781 ist die Kreuzmetathese eines Alkans mit einem Katalysator eines am Element der Gruppen 5 und 6 des periodischen Systems der Elemente auf einem festen Trägers gebundenen Alkyl-, Alkyliden- bzw. Alkylidin-Rest bekannt.

Die zuvor genannten Verfahren lassen zu wünschen übrig, da die Katalysatoren rasch desaktivieren.

Der vorliegenden Erfindung lag daher die Aufgabe zugrunde, dem zuvor genannten Nachteil abzuhelfen.

Demgemäß wurde ein neues und verbessertes Verfahren zur Herstellung von Kohlenwasserstoffen mit verändertem Kohlenstoffgerüst durch Umsetzung von aliphatischen Kohlenwasserstoffen der Formel R¹-H
a) mit sich selbst,
b) mit einem anderen aliphatischen Kohlenwasserstoff der Formel R^{1'}-H oder
c) mit aromatischen alkylsubstituierten Kohlenwasserstoffen,
wobei R¹ und R^{1'} voneinander unabhängig C₂-C₂₀ Alkylreste bedeuten, in Gegenwart eines auf einem Träger befindlichen metallorganischen Katalysators oder dessen Hydrids der allgemeinen Formel IV [Lₓ-M-R³_{n+q}]-H_{1-q}, in der
- M: ein Metall der Gruppe IIIb, IVb, Vb oder VIb des Periodensystems der Elemente ist,
- L: voneinander unabhängig ein Ligand aus der Gruppe Dialkylether, Phosphin, tert.-Amin, Halogen bedeutet,
- R³: voneinander unabhängig gesättigte oder ungesättigte Liganden mit ein bis zwanzig Kohlenstoffatomen bedeuten, die über ein oder mehrere Kohlenstoffatome an M gebunden sind,
- n: eine ganze Zahl, die kleiner oder gleich der Wertigkeit von M ist,
- q: 0 oder 1 und
- x: 0 bis n bedeuten
bei Temperaturen von 20 bis 400°C und einem Druck von 0,2 bis 100 bar gefunden, welches dadurch gekennzeichnet ist, daß man die Umsetzung in Gegenwart von Wasserstoff durchführt.

Insbesondere wurde ein neues und verbessertes Verfahren zur Herstellung von Kohlenwasserstoffen mit verändertem Kohlenstoffgerüst gefunden, welches dadurch gekennzeichnet ist, daß man Kohlenwasserstoffe mit verändertem Kohlenstoffgerüst der allgemeinen Formel I 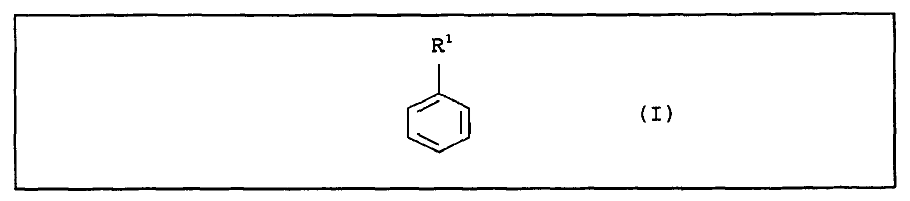 in der
- R¹: C₂- bis C₂₀-Alkyl bedeutet,
durch Umsetzung von aliphatischen Kohlenwasserstoffen der allgemeinen Formel R¹-H mit aromatischen alkylsubstituierten Kohlenwasserstoffen der allgemeinen Formel II 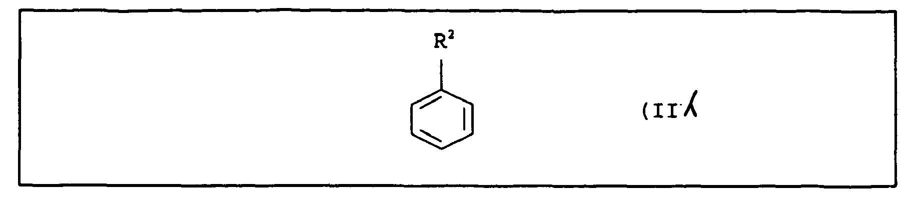 in der
- R²: C₁- bis C₄-Alkyl bedeutet, mit der Maßgabe, daß der Alkylrest von R² eine geringere oder bei unterschiedlichem Verzweigungsgrad die gleiche Anzahl an Kohlenstoffatomen enthält als der Alkylrest von R¹,
erhält.

Das erfindungsgemäße Verfahren kann wie folgt durchgeführt werden:
a) Umsetzung aliphatischer Kohlenwasserstoffe der Formel R¹-H mit sich selbst (Disproportionierung):
   Der aliphatischem Kohlenwasserstoff R¹-H und Wasserstoff können bei Temperaturen von 20 bis 400°C, bevorzugt 80 bis 350°C, besonders bevorzugt 110 bis 280°C und einem Druck von 0,2 bis 100 bar, bevorzugt 1 bis 30 bar, besonders bevorzugt 3 bis 20 bar, insbesondere 5 bis 15 bar in Gegenwart eines metallorganischen Katalysators oder dessen Hydrids IV [Lₓ-M-R³_{n+q}]-H_{1-q} umgesetzt werden.
b) Umsetzung aliphatischer Kohlenwasserstoffe der Formel R¹-H mit einem anderen aliphatischen Kohlenwasserstoff der Formel R^{1'}-H:
   Der aliphatische Kohlenwasserstoff R¹-H, ein anderer aliphatischer Kohlenwasserstoff R^{1'}-H und Wasserstoff können bei Temperaturen von 20 bis 400°C, bevorzugt 80 bis 350°C, besonders bevorzugt 110 bis 280°C und einem Druck von 0,2 bis 100 bar, bevorzugt 1 bis 30 bar, besonders bevorzugt 3 bis 20 bar, insbesondere 5 bis 15 bar in Gegenwart eines metallorganischen Katalysators oder dessen Hydrids IV [Lₓ-M-R³_{n+q}]-H_{1-q} umgesetzt werden.
   Das Molverhältnis von aliphatischem Kohlenwasserstoff R¹-H zum aliphatischem Kohlenwasserstoff R^{1'}-H kann in weiten Grenzen variieren, wobei man in der Regel im Molverhältnis von 0,7 : 1 bis 50 : 1, bevorzugt 0,8 : 1 bis 10 : 1, besonders bevorzugt 0,9 : 1 bis 3 : 1, insbesondere 1 : 1 bis 1,5 : 1 arbeitet.
c) Umsetzung aliphatischer Kohlenwasserstoffe der Formel R¹-H mit aromatischen alkylsubstituierten Kohlenwasserstoffen:
   Der aromatische alkylsubstituierte Kohlenwasserstoff II kann mit Gemischen aus aliphatischem Kohlenwasserstoff R¹-H und Wasserstoff bei Temperaturen von 20 bis 400°C, bevorzugt 80 bis 350°C, besonders bevorzugt 110 bis 280°C und einem Druck von 0,2 bis 100 bar, bevorzugt 1 bis 30 bar, besonders bevorzugt 3 bis 20 bar, insbesondere 5 bis 15 bar in Gegenwart eines metallorganischen Katalysators oder dessen Hydrids IV [Lₓ-M-R³_{n+q}]-H_{1-q} umgesetzt werden.
   Das Molverhältnis von aliphatischem Kohlenwasserstoff R¹-H zum aromatischen alkylsubstituierten Kohlenwasserstoff II kann in weiten Grenzen variieren, wobei man in der Regel im Molverhältnis von 0,7 : 1 bis 50 : 1, bevorzugt 0,8 : 1 bis 10 : 1, besonders bevorzugt 0,9 : 1 bis 3 : 1, insbesondere 1 : 1 bis 1,5 : 1 arbeitet.
   Im Falle von gasförmigen aliphatischen Kohlenwasserstoffen R¹-H kann der aromatische alkylsubstituierte Kohlenwasserstoff II bis zur Sättigung mit einem Gemisch aus aliphatischem Kohlenwasserstoff R¹-H und Wasserstoff belegt werden.

Das Molverhältnis Wasserstoff zum aliphatischen Kohlenwasserstoff R¹-H kann ebenfalls in weiten Grenzen variieren, wobei man in der Regel im Molverhältnis von 0,01 : 1 bis 100 : 1, bevorzugt 0,9 : 1 bis 30 : 1, besonders bevorzugt 1 : 1 bis 10 : 1 arbeitet. Ein größerer Überschuß an Wasserstoff ist auch möglich. Der Wasserstoff wird bevorzugt kontinuierlich während der Reaktion zugeführt. Der Wasserstoff kann in einer auf die Synthese folgenden Verfahrensstufe abgetrennt und gegebenenfalls zurückgeführt werden. Besonders bevorzugt wird mit einem möglichst geringen Anteil an Wasserstoff gearbeitet.

Die Substituenten und Indizes R¹, R², R³, M, L, n, q und x haben folgende Bedeutungen:
- R¹, R^{1'}: - mit der Maßgabe, daß R¹ und R^{1'} unterschiedlich sind C₂- bis C₂₀-Alkyl, bevorzugt C₂- bis C₁₂-Alkyl wie Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C₂- bis C₆-Alkyl wie Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl und sec.-Hexyl, insbesondere C₂- bis C₄-Alkyl wie Ethyl, n-Propyl, iso-Propyl, n-Butyl, iso-Butyl, sec.-Butyl und tert.-Butyl, ganz besonders Ethyl,
- R²: - mit der Maßgabe, daß der Alkylrest von R² eine geringere oder bei unterschiedlichem Verzweigungsgrad die gleiche Anzahl an Kohlenstoffatomen enthält als der Alkylrest von R¹
- C₁- bis C₄-Alkyl wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
- R³: - voneinander unabhängig gesättigte oder ungesättigte Liganden mit ein bis zwanzig Kohlenstoffatomen, die über ein oder mehrere Kohlenstoffatome an M gebunden sind, wie mindestens einer σ-Bindungen zwischen Kohlenstoff und M, beispielsweise C₁- bis C₂₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₅-Alkyl wie Methyl, Ethyl, n-Propyl, iso-Propyl, n-Butyl, iso-Butyl, sec.-Butyl, tert.-Butyl, n-Pentyl und Neopentyl, C₂- bis C₂₀-Alkenyl, bevorzugt C₂- bis C₈-Alkenyl wie Vinyl, Allyl, But-2-en-1-yl, But-4-en-1-yl, But-4-en-2-yl, Pent-2-en-1-yl, 2,2-Dimethyl-pent-1-en-1-yl, C₂- bis C₂₀-Alkinyl, bevorzugt C₂- bis C₈-Alkinyl wie Ethinyl, C₃- bis C₂₀-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl, C₄- bis C₂₀-Alkyl-cycloalkyl, bevorzugt C₄- bis C₁₂-Alkylcycloalkyl wie 2-Methyl-cyclopentyl, 3-Methylcyclopentyl, 2,3-Dimethyl-cyclopentyl, 2-Ethyl-cyclopentyl, 3-Ethyl-cyclopentyl, 2,3-Diethyl-cyclopentyl, 2-Ethyl-3-methyl-cyclopentyl, 3-Ethyl-2-methyl-cyclopentyl, 2-Methylcyclohexyl, 3-Methyl-cyclohexyl, 4-Methyl-cyclohexyl, C₄- bis C₂₀-Cycloalkyl-alkyl, bevorzugt C₄- bis C₁₂-Cycloalkyl-alkyl wie Cyclopentyl-methyl, Cyclohexyl-methyl, Cycloheptylmethyl, Cyclooctyl-methyl, 1-Cyclopentyl-ethyl, 1-Cyclohexylethyl, 1-Cycloheptyl-ethyl, 1-Cyclooctyl-ethyl, 2-Cyclopentyl-ethyl, 2-Cyclohexyl-ethyl, 2-Cycloheptyl-ethyl, 2-Cyclooctyl-ethyl, oder π-Bindungen zwischen Kohlenstoff und M, beispielsweise C₁- bis C₂₀-Alkylidene, bevorzugt C₁- bis C₈-Alkylidene wie Methyliden (CH₂=), Ethyliden (CH₃-CH=), Propyliden (CH₃-CH₂-CH=), Neopentyliden ([CH₃]₃-C-CH=), Allyliden (CH₂=CH-CH=), besonders bevorzugt Neopentyliden, oder C₂- bis C₂₀-Alkylidine, bevorzugt C₂- bis C₈-Alkylidine wie Ethylidin (CH₃-CH₅), Propylidin (CH₃-CH₂-CH₅), Neopentylidin ([CH₃]₃-C-CH₅), Allylidin (CH₂=CH-CH₅), besonders bevorzugt Neopentylidin, und zusätzlich gegebenenfalls C₁-bis C₂₀-Alkoxy, bevorzugt C₁- bis C₈-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy und iso-Octoxy, besonders bevorzugt C₁- bis C₅-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy und neo-Pentoxy, und Halogen wie Fluor, Chlor, Brom und Jod, bevorzugt Fluor, Chlor und Brom, besonders bevorzugt Chlor und Brom,
- M: ein Metall der Gruppe IIIb, IVb, Vb, VIb, der Lanthaniden des Periodensystems der Elemente, Magnesium, Zink, Cadmium wie Scandium, Yttrium, Lanthan, Actinium, Titan, Zirkon, Hafnium, Vanadin, Niob, Tantal, Chrom, Molybdän, Wolfram, Cer, Praseodym, Neodym, Samarium oder deren Gemische, bevorzugt Scandium, Yttrium, Lanthan, Titan, Zirkon, Hafnium, Vanadin, Niob, Tantal, Chrom, Molybdän, Wolfram, Cer oder deren Gemische, besonders bevorzugt Titan, Zirkon, Hafnium, Vanadin, Niob, Tantal, Chrom, Molybdän und Wolfram, insbesondere Zirkon, Tantal, Wolfram oder deren Gemische,
- L: voneinander unabhängig ein Ligand aus der Gruppe Dialkylether, beispielsweise C₂- bis C₂₀-Dialkylether wie Dimethylether, Ethylmethylether, Diethylether, Methylpropylether, Ethylpropylether, Dipropylether und Methyl-tert.-butylether, bevorzugt Dimethylether und Diethylether, besonders bevorzugt Dimethylether, Phosphin, beispielsweise aromatisches, aromatisch-aliphatisches oder aliphatisches Phosphin wie Trimethylphosphin, Triethylphosphin, Dimethyl-ethylphosphin, Diethylmethylphosphin, Triphenylphosphin, Dimethylphenylphosphin und Diphenylmethylphosphin, bevorzugt Trimethylphosphin, Triethylphosphin und Triphenylphosphin, besonders bevorzugt Trimethylphosphin, tert.-Amin, beispielsweise C₃- bis C₃₀-tert.-Amin wie Trimethylamin, Triethylamin, Tripropylamin und Triphenylamin, bevorzugt Trimethylamin und Triethylamin, besonders bevorzugt Trimethylamin, Halogen, beispielsweise Fluor, Chlor, Brom und Jod, bevorzugt Chlor und Brom, besonders bevorzugt Chlor,
- n: eine ganze Zahl, die kleiner oder gleich der Wertigkeit von M ist, also größer als die kleinste Oxidationsstufe von 0 bis zur maximalen Oxidationsstufe von M, die im Falle von Chrom und Niob bevorzugt 4, 5 oder 6, im Falle von Molybdän und Wolfram bevorzugt 3, 4, 5 oder 6, im Falle von Vanadin bevorzugt 4 oder 5 und im Falle von Tantal 3 oder 5 beträgt,
- q: eine ganze Zahl von 0 bis 1,
- x: eine ganze Zahl von 0 bis n, bevorzugt 0, 1 oder 2, besonders bevorzugt 0 oder 1.

Die Katalysatoren können als solche oder bevorzugt auf einem Träger eingesetzt werden. Als Träger eignen sich in der Regel anorganische Träger, insbesondere inerte Träger unter denen Oxide, Sulfide oder deren Gemische bevorzugt sind. Besonders bevorzugt sind Oxide oder deren Gemische wie Siliciumdioxid, Aluminiumoxid, Zeolithe, natürliche oder synthetische Tonerden, Aluminiumsilicate, Titandioxide, Magnesiumoxid, Nioboxid, Zirkonoxid oder deren Gemische. Insbesondere eignen sich poröse und nicht-poröse Silicalite und Aluminate, beispielsweise mesoporöse Silicalite und Aluminate mit einem mittleren Porendurchmesser von 20 bis 200Å.

Die Herstellung der metallorganischen Katalysatoren oder deren Hydride IV [Lₓ-M-R³_{n+q}]-H_{1-q} ist aus WO-A-98/02244 und WO-A-00/27781 bekannt. Bei den Hydriden IV liegt der Wasserstoff in der Regel in kovalent gebundener Form vor.

### Beispiele

Die Herstellung der in den nachfolgenden Beispielen beschriebenen Katalysatoren geht aus von Tris(neopentyl)(neopentyliden)Tantal (TaNp'Np₃), welches nach einer in J. Am. Chem. Soc. 100 (1978), Seite 3359 veröffentlichten Vorschrift zugänglich ist.

### Beispiel 1

### Herstellung des Katalysators nach dem Sublimationsverfahren

Unter Argon Schutzgasatmosphäre wurden 10 mg (TaNp'Np₃) in ein Glasgefäß eingewogen, welches mit einer "break seal" Vorrichtung ausgestattet ist. Das Glasgefäß wurde abgeschmolzen und mit einem zweiten Gefäß verbunden, in dem sich bei 500°C dehydroxyliertes SiO₂ befand. Nach Inertisierung des Reaktionsgefäßes wurde die Glasmembran zwischen den beiden Gefäßen zerbrochen und der Tantal-Komplex durch Sublimation auf dem SiO₂ Träger niedergeschlagen. Nach Abreaktion des Komplexes mit dem Träger wurde überschüssiger Komplex zurücksublimiert. Das Präparat wurde bei leichtem Unterdruck mit Wasserstoff beaufschlagt. Man erhielt ein auf SiO₂ fixiertes Ta-Hydrid. Anschließend wurde mit Hilfe der Schlenk Technik Toluol und Ethan in das Reaktionsgefäß eingeführt und die Umsetzung zu Ethylbenzol beobachtet.

Die so präparierten Proben wurden dazu verwendet, die auf der SiO₂ Oberfläche entstehenden Spezies infrarotspektroskopisch zu charakterisieren. Prinzipiell ist die Sublimationsmethode jedoch auch zur Herstellung größerer Katalysatormengen geeignet.

### Beispiel 2

### Herstellung des Katalysators: Imprägnierverfahren

Unter Luftausschluß wurden 640 mg Tris(neopentyl)neopentyliden-Tantal in trockenem (wasserfreiem) Pentan gelöst, mit 3 g bei 500°C dehydroxyliertem SiO₂ (Degussa, Aerosil, 200 m²/g) bei Raumtemperatur umgesetzt, abfiltriert und bei 150°C bei leichtem Unterdruck von 0,9 bar einer Wasserstoff-Atmosphäre ausgesetzt.

### Beispiel 3

### Kontinuierliche Umsetzung von Toluol mit Ethan in Gegenwart von Wasserstoff

Das Ethan/Wasserstoff-Gemisch wurde mit Toluol im Molverhältnis von 10 : 1 gesättigt und anschließend mit 400 mg des nach Beispiel 2 hergestellten Katalysators in einem Durchflußreaktor bei einer Temperatur von 250°C und einem Gesamtdruck von 1 bar umgesetzt. Die Reaktionsprodukte wurden gaschromatographisch auf ihre Zusammensetzung analysiert und die Ausbeute an Ethylbenzol auf die im Katalysator vorhandene Menge Tantal bezogen. Die Ergebnisse sind in Tabelle 1 zusammengestellt.

### Vergleichbeispiel A

### Kontinuierliche Umsetzung von Toluol mit Ethan ohne H₂

Es wurde wie in Beispiel 3 vorgegangen, jedoch enthielt das über den Katalysator geleitete Gasgemisch keinen Wasserstoff. Die Ergebnisse sind in Tabelle 1 zusammengestellt.

**Tabelle 1**

| Beispiel Nr. | Reaktionsd auer [h] | Umsatz/h* (relative Raten) | Ausbeute [mol Ethylbenzol/mol Tantal] |
|---|---|---|---|
| 3 | 70 | 8 | 3,4 |
| Vergleich A | 97 | 1 | 1,95 |

## Patentansprüche

1. Verfahren zur Herstellung von Kohlenwasserstoffen mit verändertem Kohlenstoffgerüst durch Umsetzung von aliphatischen Kohlenwasserstoffen der Formel R¹-H
a) mit sich selbst,
b) mit einem anderen aliphatischen Kohlenwasserstoff der Formel R^{1'}-H oder
c) mit aromatischen alkylsubstituierten Kohlenwasserstoffen,
wobei R¹ und R^{1'} voneinander unabhängig C₂-C₂₀ Alkylreste bedeuten, in Gegenwart eines auf einem Träger befindlichen metallorganischen Katalysators oder dessen Hydrids der allgemeinen Formel IV [Lₓ-M-R³_{n+q}]-H_{1-q}, in der
M ein Metall der Gruppe IIIb, IVb, Vb oder VIb des Periodensystems der Elemente ist,
L voneinander unabhängig ein Ligand aus der Gruppe Dialkylether, Phosphin, tert.-Amin, Halogen bedeutet,
R³ voneinander unabhängig gesättigte oder ungesättigte Liganden mit ein bis zwanzig Kohlenstoffatomen bedeuten, die über ein oder mehrere Kohlenstoffatome an M gebunden sind,
n eine ganze Zahl, die kleiner oder gleich der Wertigkeit von M ist,
q 0 oder 1 und
x 0 bis n bedeuten
bei Temperaturen von 20 bis 400°C und einem Druck von 0,2 bis 100 bar, **dadurch gekennzeichnet, daß** man die Umsetzung in Gegenwart von Wasserstoff durchführt.

2. Verfahren zur Herstellung von Kohlenwasserstoffen mit verändertem Kohlenstoffgerüst nach Anspruch 1, **dadurch gekennzeichnet, daß** man Kohlenwasserstoffe mit verändertem Kohlenstoffgerüst der allgemeinen Formel I 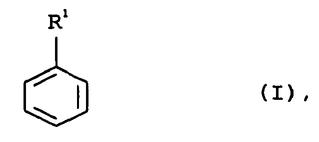 in der R¹ C₂- bis C₂₀-Alkyl bedeutet, durch Umsetzung von aliphatischen Kohlenwasserstoffen der allgemeinen Formel R¹-H mit aromatischen alkylsubstituierten Kohlenwasserstoffen der allgemeinen Formel II 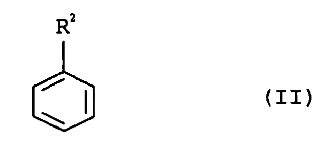 in der R² C₁- bis C₄-Alkyl bedeutet, mit der Maßgabe, daß der Alkylrest von R² eine geringere oder bei unterschiedlichem Verzweigungsgrad die gleiche Anzahl an Kohlenstoffatomen enthält als der Alkylrest von R¹, erhält.

3. Verfahren zur Herstellung von Kohlenwasserstoffen mit verändertem Kohlenstoffgerüst nach Anspruch 1, **dadurch gekennzeichnet, daß** das Molverhältnis von Wasserstoff zum aliphatischen Kohlenwasserstoff R¹-H 0,01 : 1 bis 100 : 1 beträgt.

## Claims

1. A process for preparing hydrocarbons having an altered carbon skeleton by reacting aliphatic hydrocarbons of the formula R¹-H
a) with themselves,
b) with another aliphatic hydrocarbon of the formula R¹-H or
c) with aromatic alkyl-substituted hydrocarbons,
where R¹ and R^{1'} are each, independently of one another, a C₂-C₂₀-alkyl radical, in the presence of an organometallic catalyst or hydride thereof, located on a support, of the formula IV [Lₓ-M-R³_{n+q}]-H_{1-q}, where
M is a metal of group IIIb, IVb, Vb or VIb of the Periodic Table of the Elements,
L are each, independently of one another, a ligand selected from the group consisting of diallyl ethers, phosphines, tertiary amines and halogens,
R³ are, independently of one another, saturated or unsaturated ligands having from one to twenty carbon atoms which are bound to M via one or more carbon atoms,
n is an integer less than or equal to the valence of M,
q is 0 or 1, and
x is from 0 to n,
at from 20 to 400°C and a pressure of from 0.2 to 100 bar in the presence of hydrogen.

2. The process for preparing hydrocarbons having an altered carbon skeleton according to claim 1, wherein hydrocarbons having an altered carbon skeleton of the formula I 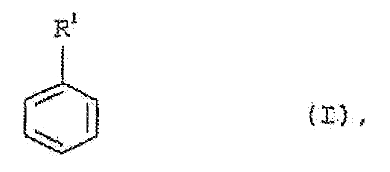 where R¹ is C₂-C₂₀-alkyl, are obtained by reacting aliphatic hydrocarbons of the formula R¹-H with aromatic alkyl-substituted hydrocarbons of the formula II 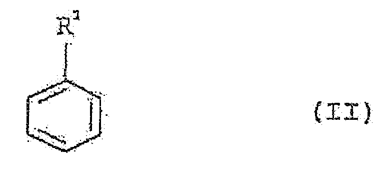 where R² is C₁-C₄-alkyl, with the proviso that the alkyl radical R² has a lower or , in the case of a different degree of branching, the same number of carbon atoms than/as the alkyl radical R¹.

3. The process for preparing hydrocarbons having an altered carbon skeleton according to claim 1, wherein the molar ratio of hydrogen to the aliphatic hydrocarbons R¹-H is from 0.01:1 to 100:1.

## Revendications

1. Procédé pour la préparation d'hydrocarbures ayant un squelette de carbone modifié par réaction d'hydrocarbures aliphatiques de la formule R¹-H :
a) avec eux-mêmes,
b) avec un autre hydrocarbure aliphatique de la formule R^{1'}-H ou
c) avec des hydrocarbures aromatiques substitués par de l'alkyle,
R¹ et R^{1'} représentant indépendamment l'un de l'autre des radicaux alkyle en C₂-C₂₀, en présence d'un catalyseur organométallique se trouvant sur un support ou de l'hydrure de celui-ci de la formule générale IV [Lₓ-N-R³_{n+q}]-H_{1-q'}, dans laquelle :
M est un métal des groupes IIIb, IVb, Vb ou VIb du système périodique des éléments,
L représente de manière indépendante un ligand issu des groupes dialkyléther, phosphine, tert-amine, halogène,
R³ représente de manière indépendante des ligands saturés ou insaturés ayant 1 à 20 atomes de carbone, qui sont liés à M par l'intermédiaire d'un ou plusieurs atomes de carbone,
n est un nombre entier inférieur ou égal à la valence de M,
q représente 0 ou 1, et
x représente 0 à n
à des températures de 20 à 400°C et à une pression de 0,2 à 100 bars, **caractérisé en ce que** la réaction est mise en oeuvre en présence d'hydrogène.

2. Procédé pour la préparation d'hydrocarbures ayant un squelette de carbone modifié selon la revendication 1, **caractérisé en ce que** des hydrocarbures ayant un squelette de carbone modifié de la formule générale I 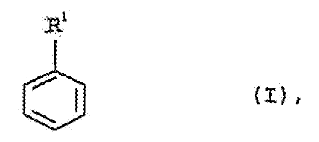 dans laquelle R¹ représente un groupe alkyle en C₂ à C₂₀, sont obtenus par réaction d'hydrocarbures aliphatiques de la formule générale R¹-H avec des hydrocarbures aromatiques substitués par de l'alkyle de la formule générale II 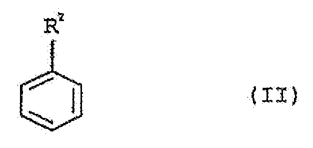 dans laquelle R² représente un groupe alkyle en Ci à C₄, sous réserve que le radical alkyle de R² contienne un nombre plus faible ou, pour un degré de ramification différent, le même nombre d'atomes de carbone que le radical alkyle de R¹.

3. Procédé pour la préparation d'hydrocarbures ayant un squelette de carbone modifié selon la revendication 1, **caractérisé en ce que** le rapport molaire de l'hydrogène sur l'hydrocarbure aliphatique R¹-H est de 0,01:1 à 100:1.
